# EUROPEAN PATENT APPLICATION

(11) **EP 1 837 010 A2**
(43) Date of publication of application: **26.09.2007**
(21) Application number: 07300864.1
(22) Date of filing: 15.03.2007
(51) Int. Cl.: A61K 8/19, A61Q 5/04

(54) **Process for relaxing or straightening hair**

(30) Priority: 17.03.2006 US 378152
(71) Applicant: L'Oréal, 75008 Paris (FR)
(72) Inventor: Cannell, David W., Plainfield, NJ 07060 (US); Nguyen, Nghi, Edison, NJ 08820 (US); Hashimoto, Sawa, Westfield, NJ 07090 (US); Espino, Cynthia, Princeton, NJ 08854 (US)
(74) Representative: Martin-Charbonneau, Virginie

(57) **Abstract**

A process for straightening or relaxing hair involving the steps of: (a) contacting the hair with a hair straightening/relaxing composition containing from 0.01 to 2% by weight of at least one hydroxide compound to form treated hair; (b) optionally, rinsing the hair straightening/relaxing composition from the treated hair, after it has been in contact with the hair for a period of less than 60 minutes; (c) optionally, contacting the treated hair with a non-volatile oil chosen from plant, animal, mineral and synthetic oils; and (d) smoothing the treated hair using a combination of heat and means for physically smoothing hair.

## Description

Hair straightening or hair relaxing products have been commercially available for over fifty years for people who want straighter, more manageable hair. Most commercially available hair relaxers are composed of a strong hydroxide base that breaks the bonds in the hair.

Commercial products based only on alkaline metal hydroxides such as sodium hydroxide and lithium hydroxide are typically used to straighten or relax curly/kinky hair. There are primarily four different types of alkaline metal hydroxide hair straighteners in use: calcium hydroxide, lithium hydroxide, sodium hydroxide, and potassium hydroxide. The straightening product is usually applied quickly and can only remain on the hair for a very limited amount of time. Due to the alkalinity of such products, if the product is not rinsed from the hair at the appropriate time damage to the hair can occur, as well as chemical burns to the scalp and areas surrounding the hair.

Thus, the object of the present invention is to provide a hair straightening or relaxing process which is safer, yet just as effective as, conventional processes.

The present invention is directed to a process for straightening or relaxing hair involving the steps of:

(a) providing a hair straightening/relaxing composition containing:
   i. from 0.01% to 2% by weight of at least one hydroxide compound; and
   ii. remainder, to 100%, a cosmetically acceptable medium;
      all weights based on the total weight of the hair straightening/relaxing composition;
(b) contacting the hair with the hair straightening/relaxing composition to form treated hair;
(c) optionally, rinsing the hair straightening/relaxing composition from the treated hair;
(d) optionally, contacting the treated hair with a non-volatile oil; and
(e) smoothing the treated hair using a combination of heat and means for physically smoothing hair; steps (d) and (e) being performed prior to and/or after steps (a) to (c).

Other than in the operating examples, or where otherwise indicated, all numbers expressing quantities of ingredients and/or reaction conditions, are to be understood as being modified in all instances by the term "about".

The present invention is directed to a process for straightening or relaxing hair using at least: (a) a straightening/relaxing composition containing: (i) at least one hydroxide compound and (ii) a cosmetically acceptable medium, and (b) means for ironing hair.

It has been surprisingly found that by employing the process of the present invention, straightening/relaxing of the hair can be achieved in a manner which is safe for the user's skin, as well as their hair. This is due to the present invention's use of low amounts of hydroxide in the straightening/relaxing process. Conventional products, which employ larger amounts of hydroxide, have a tendency to cause both skin irritation, as well as damage to the hair, due to the presence of large amounts of hydroxide in said products. However, by employing a low hydroxide-containing product, in combination with heat and means for physically smoothing the hair, satisfactory straightening/relaxing of the hair can be achieved in a manner that is safe for both skin and hair.

Suitable hydroxide compounds for use in the present invention may be chosen, for example, from alkali metal hydroxides, alkaline-earth metal hydroxides, transition metal hydroxides, and organic hydroxides, such as for example sodium hydroxide, potassium hydroxide, lithium hydroxide, rubidium hydroxide, caesium hydroxide, francium hydroxide, beryllium hydroxide, magnesium hydroxide, calcium hydroxide, strontium hydroxide, barium hydroxide, molybdenum hydroxide, manganese hydroxide, zinc hydroxide, cobalt hydroxide, cadmium hydroxide, cerium hydroxide, lanthanum hydroxide, actinium hydroxide, thorium hydroxide, aluminium hydroxide, guanidinium hydroxide and quaternary ammonium hydroxides, and mixtures thereof.

A particularly preferred hydroxide compound for use in the present invention is sodium hydroxide.

The hydroxide compound is employed in the hair straightening/relaxing composition in an amount of from 0.01% to 2% by weight, preferably from 0.2% to 1.5% by weight, preferably from 0.5% to 1% by weight, based on the total weight of the composition.

As used herein, the term "cosmetically acceptable medium" is known to one of ordinary skill in the art, and may comprise, for example, water and/or at least one organic solvent.

The hair straightening/relaxing composition disclosed herein may be, for example, in the form of a thickened cream so as to hold the hair as stiff as possible. These creams are made for example in the form of "heavy" emulsions, containing water and at least one oil chosen preferably from glyceryl stearate, glycol stearate, self-emulsifying waxes, fatty alcohols, mineral oil and petrolatum, and mixtures thereof.

The hair straightening/relaxing composition may contain thickeners, such as carboxyvinyl polymers or copolymers that "stick" the hairs together and hold them in a smooth position during the leave-in time, may also be used. It may for example be in the form of a liquid or a gel containing suc a thickener.

The hair straightening/relaxing composition as disclosed herein may also comprise at least one adjuvant chosen, for example, from silicones in soluble, dispersed and microdispersed forms, nonionic, anionic, cationic and amphoteric surfactants, ceramides, glycoceramides and pseudoceramides, vitamins and provitamins including panthenol, waxes other than ceramides, glycoceramides and pseudoceramides, water-soluble and liposoluble, silicone-based and non- silicone-based sunscreens, nacreous agents and opacifiers, sequestering agents, plasticizers, solubilizers, acidifying agents, mineral and organic thickeners, antioxidants, hydroxy acids, penetrating agents, fragrances, and preserving agents.

Smoothing of hair treated with the above-disclosed hair straightening/relaxing composition involves using a combination of heat and means for physically smoothing the hair. The heat necessary to effectuate smoothing should be preferably at least 50°C, more preferably at least 75°C, even more preferably at least 100°C. The precise amount of heat employed will depend on the concentration of hydroxide compound present in the composition. This heat may emanate from any suitable source such as, for example, a hair dryer or hot/flat iron.

The means for physically smoothing hair can be any apparatus capable of physically smoothing the hair such as, for example, a hair brush or a comb. In one embodiment, the means for smoothing hair also serves as the source for generating heat such as, for example, a hot/flat iron.

According to one embodiment of the present invention, there is provided a process for straightening or relaxing hair involving the steps of: (a) contacting the hair with the above-disclosed hair straightening/relaxing composition to form treated hair; (b) optionally, rinsing the hair straightening/relaxing composition from the treated hair, after it has been in contact with the hair for a period of less than 60 minutes, preferably less than 40 minutes, more preferably less than 30 minutes; (c) optionally, contacting the treated hair with a non-volatile oil chosen from plant, animal, mineral and synthetic oils, and mixtures thereof; and (d) smoothing the treated hair using a combination of heat and means for physically smoothing hair.

As is disclosed above, the hair straightening/relaxing composition may either be left on the hair, or rinsed off. As for the non-volatile oil, if employed, it will preferably remain on the hair.

According to another embodiment of the present invention, there is provided a process for straightening or relaxing hair involving the steps of: (a) optionally, contacting the hair with a non-volatile oil chosen from plant, animal, mineral and synthetic oils, and mixtures thereof; (b) smoothing the treated hair using a combination of heat and means for physically smoothing hair to form smoothed hair; (c) contacting the smoothed hair with the hair straightening/relaxing composition to form treated hair; (d) optionally, rinsing the hair straightening/relaxing composition from the treated hair after it has been in contact with the hair for a period of less than 60 minutes, preferably less than 40 minutes, preferably less than 30 minutes; and (e) optionally, smoothing the treated hair using a combination of heat and means for physically smoothing hair to form smoothed hair.

It should be noted that the steps of: contacting the hair with a non-volatile oil; and smoothing the hair, may be performed prior to and/or after application of the hair straightening/relaxing composition. Preferably, they are performed after application of the hair straightening/relaxing composition, and after the rinsing step if any.

The present invention also concerns the use of a hair straightening/relaxing composition as defined above in combination with means for heating the hair, for straightening or relaxing human hair

In commercially available hair straightening or relaxing compositions, higher concentrations of hydroxide compound must be used in order to satisfactorily straighten/relax the hair without heat. In the present invention, however, lower concentrations of hydroxide compound can be used because of the synergy realized by using a combination of heat and an apparatus capable of physically smoothing the hair. Without intending to be bound by theory, it is believed that a synergistic effect in hair straightening/relaxing is realized due to an induced supercontraction and denaturation of hair protein caused by the combination of heat and physical smoothing with the low hydroxide hair straightening/relaxing composition.

Moreover, due to the lower concentrations of hydroxide compound being used, a barrier substance is not required when using the hair straightening/relaxing composition of the present invention. Commercially available hair relaxing products oftentimes require the hair stylist to apply a barrier substance such as petrolatum to the skin surrounding the scalp and the area around the ears. The barrier substance is used to prevent the skin from becoming irritated if the hair relaxing product contacts the skin. A barrier substance is not necessary when using the process of the present invention because the concentration of hydroxide compound is much lower.

The present invention will be better understood from the examples which follow, all of which are intended for illustrative purposes only, and are not meant to unduly limit the scope of the invention in any way.

EXAMPLES

General Procedure to Test the Straightening Efficiency of Kinky Hair.

The tests were done on hair swatches made of 45 strands of kinky hair, 10 cm long (when straight). The following treatments were performed:

Treat: The hair swatches were treated with NaOH by being soaked in the various aqueous NaOH solutions (ie solutions consisting of water and NaOH). The kinky hair swatches were soaked in a straight configuration (the hair was stretched on a paper board) for the indicated amount of time, then the treated hair swatches were removed from the paper board.

Leave-on: The treated hair swatches were blotted dry once with the paper towel then blow-dried at 55°C - 65°C until completely dry.

Rinse Out: The treated hair swatches were rinsed with water for 5 seconds then blow-dried at 55°C - 65°C until completely dry.

Heat: The hair was then straightened by passing the flat iron at 193°C over the hair three times, 6-7 seconds for each pass.

After the treatments, as defined above, the straightened hair swatches were shampooed three times, using 10% SLES-2 (pH=6.0) solution, for 5 seconds of shampooing and 5 seconds of rinsing each time. The final length of the hair was measured when the hair was completely dry at ambient condition.

The % Straightening Efficiency (SE) was calculated using the following formula: % straightening efficiency = (A/B) x 100, where A = final length measured (cm), B=initial length of hair (10cm).

The following examples show the percent Straightening Efficiency of hair treated with various protocols.

Example 1. Treat - Leave-on - Heat

The kinky hair was treated with 0.05%, 0.10%, 0.15%, 0.25%, 0.50%, 1.0% NaOH solution for 5 minutes or 30 minutes as leave-on treatments then flat ironed three times. The % Straightening Efficiencies (%SE) are shown below.

| | | | | | | | | |
|---|---|---|---|---|---|---|---|---|
| NaOH: | 0.05% | 0.10% | 0.15% | 0.25% | 0.5% | 1.0% | Soak | Iron 3x |
| %SE: | | | | | | | | |
| | 30% | 31% | 32% | 31% | 38% | 52% | 30 min | No |
| | 50% | 60% | 68% | 96% | 97% | 98% | 30 min | Yes |
| | 37% | 45% | 45% | 50% | 75% | 85% | 5 min | Yes |

The results show that in the Treat - Leave-on - Heat protocol, the use of heat improved the straightening efficiency.

Example 2. Heat - Treat - Leave-on

The kinky hair was heated with the flat iron three times then treated with 0.05%, 0.10%, 0.15%, 0.25%, 0.50%, 1.0% NaOH solution for 30 minutes as leave-on treatments. The % Straightening Efficiencies (SE) are shown below.

| | | | | | | | |
|---|---|---|---|---|---|---|---|
| NaOH: | 0.05% | 0.10% | 0.15% | 0.25% | 0.50% | 1.00% | Iron 3x |
| % SE: | | | | | | | |
| | 30% | 31% | 32% | 31% | 38% | 52% | No |
| | 43% | 45% | 46% | 50% | 52% | 60% | Yes |

The results show that in the Heat - Treat - Leave-on protocol, the use of heat improved the straightening efficiency.

Example 3. Treat - Rinse-off - Heat

The kinky hair was treated with 0.05%, 0.10%, 0.15%, 0.25%, 0.50%, 1.0% NaOH solution for 5 minutes or 30 minutes as rinse-off treatments then flat ironed three times. The % Straightening Efficiencies (SE) are shown below.

| | | | | | | | | |
|---|---|---|---|---|---|---|---|---|
| NaOH: | 0.05% | 0.10% | 0.15% | 0.25% | 0.50% | 1.00% | Soak | Iron 3x |
| %SE: | | | | | | | | |
| | 30% | 30% | 30% | 30% | 40% | 50% | 30 min | No |
| | 33% | 40% | 50% | 70% | 80% | 90% | 30 min | Yes |
| | 35% | 35% | 40% | 40% | 63% | 87% | 5 min | Yes |

The results show that in the Treat - Rinse-off - Heat protocol, the use of heat improved the straightening efficiency.

Example 4. Heat - Treat - Leave-on - Heat

The kinky hair was flat ironed three times then treated with 0.05%, 0.10%, 0.15% NaOH solution for 5 minutes or 30 minutes as leave-on treatments then flat ironed three times again. The % Straightening Efficiencies (SE) are shown below.

| | | | | | | |
|---|---|---|---|---|---|---|
| NaOH: | 0.05% | 0.10% | 0.15% | Iron 3x | Soak | Iron 3x |
| %SE: | | | | | | |
| | 30% | 31% | 32% | No | 30 min | No |
| | 75% | 88% | 92% | Yes | 30 min | Yes |
| | 48% | 50% | 60% | Yes | 5 min | Yes |

The results show that in the Heat - Treat - Leave-on - Heat protocol, the use of heat improved the straightening efficiency.

Example 5. Heat - Treat - Rinse-off - Heat

The kinky hair was flat ironed three times then treated with 0.05%, 0.10%, 0.15% NaOH solutions for 5 minutes or 30 minutes as rinse-off treatments then flat ironed three times again. The % Straightening Efficiencies (SE) are shown below.

| | | | | | | |
|---|---|---|---|---|---|---|
| NaOH: | 0.05% | 0.10% | 0.15% | Iron 3x | Soak | Iron 3x |
| %SE: | | | | | | |
| | 30% | 30% | 30% | No | 30 min | No |
| | 47% | 52% | 72% | Yes | 30 min | Yes |
| | 40% | 40% | 50% | Yes | 5 min | Yes |

The results show that in the Heat - Treat - Rinse-off - Heat protocol, the use of heat improved the straightening efficiency.

As can be seen from the above data, the use of a hot iron significantly increases the degree of straightening achieved by the hair straightening/relaxing composition of the present invention.

## Claims

1. A process for straightening or relaxing hair, comprising:
(a) providing a hair straightening/relaxing composition containing:
(i) from 0.01 to 2% by weight of at least one hydroxide compound; and
(ii) remainder, to 100%, a cosmetically acceptable medium,
all weights based on the total weight of the hair straightening/relaxing composition;
(b) contacting the hair with the hair straightening/relaxing composition to form treated hair;
(c) optionally, rinsing the hair straightening/relaxing composition from the treated hair;
(d) optionally, contacting the treated hair with a non-volatile oil;
(e) smoothing the treated hair using a combination of heat and means for physically smoothing hair;
steps (d) and (e) being performed prior to and/or after steps (a) to (c).

2. The process of claim 1, wherein said hydroxide compound is employed in an amount of from 0.2 to 1.5% by weight, based on the total weight of the hair straightening/relaxing composition.

3. The process of claim 2, wherein said hydroxide compound is employed in an amount of from 0.5 to 1% by weight, based on the total weight of the hair straightening/relaxing composition.

4. The process of any preceding claim, wherein said hydroxide compound is chosen from alkali metal hydroxides, alkaline-earth metal hydroxides, transition metal hydroxides, organic hydroxides, and mixtures thereof.

5. The process of the preceding claim, wherein said hydroxide compound is chosen from sodium hydroxide, potassium hydroxide, lithium hydroxide, rubidium hydroxide, caesium hydroxide, francium hydroxide, beryllium hydroxide, magnesium hydroxide, calcium hydroxide, strontium hydroxide, barium hydroxide, molybdenum hydroxide, manganese hydroxide, zinc hydroxide, cobalt hydroxide, cadmium hydroxide, cerium hydroxide, lanthanum hydroxide, actinium hydroxide, thorium hydroxide, aluminium hydroxide, guanidinium hydroxide and quaternary ammonium hydroxides, and mixtures thereof.

6. The process of the preceding claim, wherein said hydroxide compound is sodium hydroxide.

7. The process of any preceding claim, wherein step (b) is performed for less than 60 minutes.

8. The process of the preceding claim , wherein step (b) is performed for less than 30 minutes.

9. The process of the preceding claim, wherein step (b) is performed for less than 20 minutes.

10. The process of any preceding claim, wherein the process is performed without the use of a barrier substance.

11. The process of any preceding claim wherein said non-volatile oil is chosen from plant, mineral, animal and synthetic oils, and mixtures thereof.

12. The process of any preceding claim wherein the heat employed in step (e) is at least 50°C.

13. The process of the preceding claim, wherein the heat employed in step (e) is at least 100°C.

14. The process of any preceding claim, wherein the means for physically smoothing hair is chosen from a brush and a comb.

15. The process of any preceding claim wherein step (e) is performed using a hot/flat iron at a temperature of at least 100°C.

16. The use, for straightening or relaxing human hair, of a composition comprising, in a cosmetically acceptable medium, from 0.01 to 2% by weight based on the total weight of the composition of at least one hydroxide compound, in combination with means for heating the hair.
